# FASCICULE DE BREVET EUROPEEN

(11) **EP 1 324 976 B1**
(45) Date de publication et mention de la délivrance du brevet: **01.02.2006**
(21) Numéro de dépôt: 01976353.1
(22) Date de dépôt: 03.10.2001
(51) Int. Cl.: C07C 253/10, C07C 253/30, C07C 255/07

(54) **PROCEDE D'HYDROCYANATION DE COMPOSES ORGANIQUES A INSATURATION ETHYLENIQUE**
VERFAHREN ZUR HYDROCYANIERUNG VON ÄTHYLENISCH UNGESÄTTIGTEN ORGANISCHEN VERBINDUNGEN
HYDROCYANATION METHOD FOR ETHYLENICALLY UNSATURATED ORGANIC COMPOUNDS

(30) Priorité: 13.10.2000 FR 0013152
(43) Date de publication de la demande: 09.07.2003
(73) Titulaire: RHODIA POLYAMIDE INTERMEDIATES, 69192 Saint-Fons (FR)
(72) Inventeur: BURATTIN, Paolo, 68400 Riedishiem (FR); GALLAND, Jean-Christophe, F-69003 LYON (FR); CHAMARD, Alex, F-69960 CORBAS (FR)
(74) Mandataire: Esson, Jean-Pierre
(86) Numéro de dépôt international: PCT/FR2001/003047
(87) Numéro de publication internationale: WO 2002/030854

(56) Documents cités:
- WO-A-00/39134
- FR-A- 2 588 197
- FR-A- 2 785 610

## Description

La présente invention concerne un procédé d'hydrocyanation de composés organiques à insaturation éthylénique en composés comprenant au moins une fonction nitrile.

Elle se rapporte plus particulièrement à l'hydrocyanation de dioléfines telles que le butadiène ou d'oléfines substituées telles que des alcènesnitriles comme les pentènes nitriles.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'un phosphite de triaryle. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

Le brevet FR-A-2 338 253 a proposé de réaliser l'hydrocyanation des composés ayant au moins une insaturation éthylénique, en présence d'une solution aqueuse d'un composé d'un métal de transition , notamment le nickel, le palladium ou le fer, et d'une phosphine sulfonée.

Les phosphines sulfonées décrites dans ce brevet sont des triarylphosphines sulfonées et plus particulièrement des triphénylphosphines sulfonées.

Ce procédé permet une hydrocyanation correcte, notamment du butadiène et des pentène-nitriles, une séparation aisée de la solution catalytique par simple décantation et par conséquent évite au maximum le rejet d'effluents ou de déchets contenant les métaux servant de catalyseur. Le brevet WO00/39134 décrit des réactions organiques catalysées par un système biphasique comprenant un ligand organophosphoré sultané traité pour éliminer les sulfites.

Toutefois, des recherches sont conduites pour trouver de nouveaux systèmes catalytiques plus performants tant en activité catalytique qu'en stabilité.

Un des buts de la présente invention est de proposer une nouvelle famille de ligands qui permet d'obtenir avec les métaux de transition des systèmes catalytiques présentant une activité améliorée par rapport aux systèmes connus.

A cet effet, l'invention propose un procédé d'hydrocyanation de composés organiques comprenant au moins une liaison éthylénique par réaction avec le cyanure d'hydrogène, en présence d'un système catalytique comprenant un métal de transition et un ligand organophosphoré, caractérisé en ce que le ligand est une phosphine répondant à la formule générale (I) suivante : 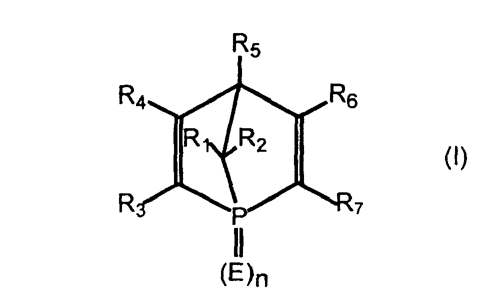
dans laquelle :
E représente O ou S ;
n représente 0 ou 1 ;
R₁, R₄, R₅ et R₆, identiques ou différents, représentent un atome d'hydrogène; ou bien un radical T choisi parmi un radical hydrocarboné aliphatique, de 1 à 40 atomes de carbone, saturé ou insaturé, dont la chaîne hydrocarbonée est éventuellement interrompue par un hétéroatome; un radical carbocyclique ou hétérocyclique, saturé, insaturé ou aromatique, monocyclique ou polycyclique, ou un radical hydrocarboné aliphatique saturé ou insaturé dont la chaîne hydrocarbonée est éventuellement interrompue par un hétéroatome et porteur d'un radical carbocyclique ou hétérocyclique tel que défini ci-dessus,
ou bien R₄ et R₅ forment ensemble avec les atomes de carbone qui les portent un monocycle carbocyclique saturé ou insaturé ayant de préférence de 5 à 7 atomes de carbone ;
R₂ représente un atome d'hydrogène ou le radical X ;
R₃ représente le radical X ou le radical Y ;
étant entendu qu'un et un seul des substituants R₂ et R₃ représente le radical X ;
X étant choisi parmi un radical carbocyclique ou hétérocyclique aromatique monocyclique ou bicyclique ayant de 2 à 20 atomes de carbone ; un radical 1-alcényle présentant éventuellement une ou plusieurs insaturations supplémentaires dans la chaîne hydrocarbonée et ayant de 2 à 12 atomes de carbone ; un radical 1-alcynyle présentant éventuellement une ou plusieurs insaturations supplémentaires dans la chaîne hydrocarbonée et ayant de 2 à 12 atomes de carbone ; un radical -CN ; [(C₁-C₁₂)alkyl]carbonyle ; [(C₃₋C₁₈)aryl]carbonyle ; [(C₁-C₁₂)alcoxy]carbonyle ; [(C₆-C₁₈)aryloxy]carbonyle ; carbamoyle ; [(C₁-C₁₂)alkyl]carbamoyle ; ou [di(C₁-C₁₂)alkyl]carbamoyle; et
Y prenant l'une quelconque des significations de R₁; à l'exception de l'atome d'hydrogène
R₇ a la signification de R₁, R₄, R₅ et R₆ ou représente un radical hydrocarboné comprenant une fonction carbonyle ou un radical de formules suivantes : 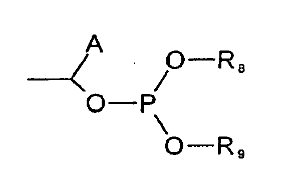
   ou

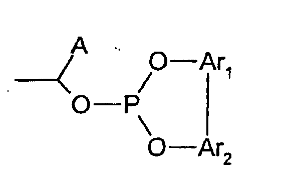

dans lesquelles,
A représente un atome d'hydrogène ; (C₁-C₁₀)alkyle ; ou (C₆-C₁₀)aryle ou (C₆-C₁₀)aryl-(C₁-C₁₀)alkyle dans lesquels la partie aryle est éventuellement substituée par un ou plusieurs radicaux choisis parmi (C₁-C₆)alkyle, (C₁₋C₆)alcoxy, trifluorométhyle, halogène, di(C₁-C₆)alkylamino, (C₁₋C₆)alcoxycarbonyle, carbamoyle, (C₁-C₆)alkylaminocarbonyle et di(C₁₋C₆)alkylaminocarbonyle ;
-Ar₁; Ar₂ représentent :
- soit le radical divalent de formule : 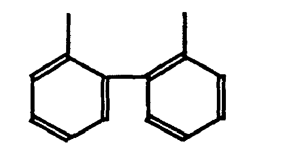
   dans lequel chacun des noyaux phényle est éventuellement substitué par un ou plusieurs groupes Z tels que définis ci-dessous :
- soit le radical divalent de formule : 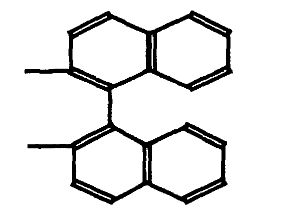
   dans lequel chacun des noyaux phényle est éventuellement substitué par un ou plusieurs groupes Z tels que définis ci-dessous

Z représente (C₁-C₆)alkyle, (C₁-C₆)alcoxy, trifluorométhyle, halogène, (C₁₋C₆)alcoxycarbonyle, di (C₁-C₆)alkylamino, (C₁-C₆)alkylaminocarbonyle ou di(C₁₋C₆)alkylaminocarbonyle.
R₈ et R₉ identiques ou différents représentent un radical aryle substitué ou non. Cette famille de composés phosphines est décrite dans les demandes de brevet français n°2 785 610 et 2 785 611. Des exemples de procédé de fabrication de ces composés sont également décrits dans les documents précités.

Parmi les composés décrits, les composés répondant à la formule (I) dans laquelle :
R₁, R₄, R₅ et R₆ représentent indépendamment un atome d'hydrogène ou bien un radical T choisi parmi :
   · un radical hydrocarboné aliphatique, saturé ou insaturé, ayant de 1 à 12 atomes de carbone, dont la chaîne hydrocarbonée est éventuellement interrompue par un hétéroatome choisis parmi O, N et S;
   · un radical carbocyclique monocyclique saturé ou comprenant 1 ou 2 insaturations dans le cycle, ayant de 3 à 8 atomes de carbone;
   · un radical carbocyclique bicyclique saturé ou insaturé constitué de 2 monocycles condensés l'un à l'autre, chaque monocycle comprenant éventuellement 1 à 2 insaturations et présentant de 3 à 8 atomes de carbone;
   · un radical carbocyclique aromatique mono- ou bicyclique en C₆-C₁₀;
   · un radical hétérocyclique monocyclique saturé, insaturé ou aromatique de 5 à 6 chaînons comprenant 1 à 3 hétéroatomes choisis indépendamment parmi N, O et S;
   · un radical hétérocyclique bicyclique saturé, insaturé ou aromatique constitué de deux monocycles de 5 à 6 chaînons condensés l'un à l'autre, chaque monocycle comprenant 1 à 3 hétéroatomes choisis indépendamment parmi O, N et S; et
   · un radical hydrocarboné aliphatique saturé ou insaturé, ayant de 1 à 12 atomes de carbone, dont la chaîne hydrocarbonée porte un radical monocyclique carbocyclique ou hétérocyclique tel que défini ci-dessus,
R2 représente un atome d'hydrogène ou bien un radical X choisi parmi :
   · un radical carbocyclique monocyclique saturé ou comprenant 1 ou 2 insaturations dans le cycle, ayant de 3 à 8 atomes de carbone;
   · un radical carbocyclique bicyclique saturé ou insaturé constitué de 2 monocycles condensés l'un à l'autre, chaque monocycle comprenant éventuellement 1 à 2 insaturations et présentant de 3 à 8 atomes de carbone;
   · un radical carbocyclique aromatique mono- ou bicyclique en C₆-C₁₀;

De préférence, X est choisi parmi un groupe alcényle en (C₂-C₆), un groupe alcynyle en (C₂-C₆), phényle, naphtyle, thiényle, furyle, pyrrolyle, imidazolyle, pyrazolyle, thiazolyle, oxazolyle, pyridyle, pyrazinyle, pyridazinyle, isothiazolyle, isoxazolyle, benzofuryle, benzothiényle, indolyle, isoindolyle, indolizinyle, indazolyle, purinyle, quinolyle, isoquinolyle, benzoxazolyle, benzothiazolyle et ptéridinyle.
Comme composés préférés de l'invention, on peut citer les composés de formules (II) ou (III) suivantes :
Formule (II) : 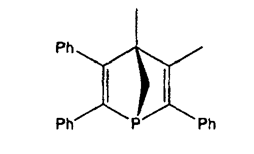
Formule III

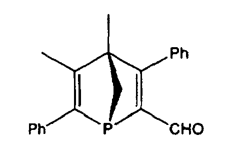

Comme métal de transition, les composés des métaux de transition, plus particulièrement les composés du nickel, du palladium et du fer sont utilisés.

Parmi les composés précités, les composés les plus préférés sont ceux du nickel, On peut citer à titre d'exemples non limitatifs :
- les composés dans lesquel le nickel est au degré d'oxydation zéro comme lé tétracyanonickelate de potassium K₄ [Ni(CN)₄], le bis (acrylonitrile) nickel zéro, le bis (cyclooctadiène-1,5) nickel et les dérivés contenant des ligands du groupe Va comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs le BH₄Na, la poudre de Zn, le magnésium, le BH₄K et les borohydrures

Quand le composé du nickel utilisé correspond à l'état d'oxydation 0 du nickel, on peut également ajouter un réducteur du type de ceux précités, mais cet ajout n'est pas impératif.

Quand on utilise un composé du fer, les mêmes réducteurs conviennent.

Dans le cas du palladium, les réducteurs peuvent être, en outre, des éléments du milieu réactionnel (phosphine, solvant, oléfine).

Les composés organiques comportant au moins une double liaison éthylénique plus particulièrement mis en oeuvre dans le présent procédé sont les dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3 nitrile, le pentène-4 nitrile, les monooléfines comme le styrène, le méthyl-styrène, le vinyl-naphtalène, le cyclohexène, le méthyl-cyclohexène ainsi que les mélanges de plusieurs de ces composés.

Les pentène-nitriles notamment peuvent contenir des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène, provenant par exemple de la réaction antérieure d'hydrocyanation du butadiène en nitriles insaturés.

En effet, lors de l'hydrocyanation du butadiène, il se forme avec les pentène-nitriles linéaires des quantités non négligeables de méthyl-2 butène-3 nitrile et de méthyl-2 butène-2 nitrile.

Le système catalytique utilisé pour l'hydrocyanation selon le procédé de l'invention peut être préparé avant son introduction dans la zone de réaction, par exemple par addition à la phosphine de formule (I) seule ou dissoute dans un solvant, de la quantité appropriée de composé du métal de transition choisi et éventuellement du réducteur. Il est également possible de préparer le système catalytique "in situ" par simple addition de la phosphine et du composé du métal de transition dans le milieu réactionnel d'hydrocyanation avant ou après l'addition du composé à hydrocyaner.

La quantité de composé du nickel ou d'un autre métal de transition utilisée est choisie pour obtenir une concentration en mole de métal de transition par mole de composés organiques à hydrocyaner ou isomériser comprise entre 10⁻⁴ et 1, et de préférence entre 0,005 et 0,5 mole de nickel ou de l'autre métal de transition mis en oeuvre.

La quantité de phosphine de formule (I) utilisée est choisie de telle sorte que le nombre de moles de ce composé rapporté à 1 mole de métal de transition soit de 0,5 à 500 et de préférence de 2 à 100.

Bien que la réaction soit conduite généralement sans solvant, il peut être avantageux de rajouter un solvant organique inerte.

A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone.

Le cyanure d'hydrogène est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que la phosphine, le composé de métal de transition, les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés, par exemple, par distillation.

Un perfectionnement au procédé d'hydrocyanation de composés à insaturation éthylénique selon la présente invention concerne notamment l'hydrocyanation desdits composés nitriles à insaturation éthylénique, par réaction avec le cyanure d'hydrogène et consiste à utiliser un système catalytique conforme à la présente invention avec un cocatalyseur consistant en au moins un acide de Lewis.

Les composés à insaturation éthylénique qui peuvent être mis en oeuvre dans ce perfectionnement sont de manière générale ceux qui ont été cités pour le procédé de base. Cependant il est plus particulièrement avantageux de l'appliquer à la réaction d'hydrocyanation en dinitriles des nitriles aliphatiques à insaturation éthylénique, notamment aux pentène-nitriles linéaires comme le pentène-3 nitrile, le pentène-4 nitrile et leurs mélanges.

Ces pentène-nitriles peuvent contenir des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène, provenant de la réaction antérieure d'hydrocyanation du butadiène et/ou de l'isomérisation du méthyl-2 butène-3 nitrile en pentène-nitriles.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments. Ces composés sont le plus souvent des sels, notamment des halogénures, comme chlorures ou bromures, sulfates, sulfonates, halogenosulfonates, perhalogénoalkyl sulfonates, notamment fluoroalkylsulfonates ou perfluoroalkylsulfonates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium, le chlorure de cobalt, le chlorure ferreux, le chlorure d'yttrium.

On peut bien entendu mettre en oeuvre des mélanges de plusieurs acides de Lewis.

Parmi les acides de Lewis, on préfére tout particulièrement le chlorure de zinc, le bromure de zinc, le chlorure stanneux, le bromure stanneux et les mélanges chlorure de zinc/chlorure stanneux.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

Comme pour la mise en oeuvre du procédé de base de l'invention, la solution catalytique utilisée pour l'hydrocyanation en présence d'acide de Lewis peut être préparée avant son introduction dans la zone de réaction, par exemple par addition au milieu réactionnel de la phosphine de formule (I), de la quantité appropriée de composé du métal de transition choisi, de l'acide de Lewis et éventuellement du réducteur. Il est également possible de préparer la solution catalytique "in situ" par simple mélange de ces divers constituants.

Il est également possible dans les conditions du procédé d'hydrocyanation de la présente invention, et notamment en opérant en présence du catalyseur décrit précédemment comportant au moins une phosphine de formule (I) et au moins un composé d'un métal de transition, de réaliser, en absence de cyanure d'hydrogène, l'isomérisation du méthyl-2-butène-3-nitrile en pentène-nitriles, et plus généralement des nitriles insaturés ramifiés en nitriles insaturés linéaires.

Le méthyl-2-butène-3-nitrile soumis à l'isomérisation selon l'invention peut être mis en oeuvre seul ou en mélange avec d'autres composés.

Ainsi on peut engager du méthyl-2-butène-3-nitrile en mélange avec du méthyl-2 butène-2 nitrile, du pentène-4 nitrile, du pentène-3 nitrile, du pentène-2 nitrile, du butadiène, de l'adiponitrile, du méthyl-2 glutaronitrile, de l'éthyl-2 succinonitrile ou du valéronitrile.

il est particulièrement intéressant de traiter le mélange réactionnel provenant de l'hydrocyanation du butadiène par HCN en présence d'au moins une phosphine de formule (I) et d'au moins un composé d'un métal de transition, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10°C à 200°C et de préférence de 60°C à 120°C.

Dans le cas préféré d'une isomérisation suivant immédiatement la réaction d'hydrocyanation du butadiène, il sera avantageux d'opérer à la température à laquelle l'hydrocyanation a été conduite.

Comme pour le procédé d'hydrocyanation de composés à insaturation éthylénique, le système catalytique utilisé pour l'isomérisation peut être préparé avant son introduction dans la zone de réaction, par exemple par addition dans le milieu réactionnel de la phosphine de formule (I), de la quantité appropriée de composé du métal de transition choisi et éventuellement du réducteur. Il est également possible de préparer le système catalytique "in situ" par simple mélange de ces divers constituants. La quantité de composé du métal de transition et plus particulièrement du nickel utilisée, ainsi que la quantité de phosphine de formule (I) sont les mêmes que pour la réaction d'hydrocyanation.

Bien que la réaction d'isomérisation soit conduite généralement sans solvant, il peut être avantageux de rajouter un solvant organique inerte qui pourra être celui de l'extraction ultérieure. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus, la réaction d'hydrocyanation des nitriles insaturés en dinitriles pouvant être mis en oeuvre avec un système catalytique conforme à l'invention ou tout autre système catalytique déjà connu pour cette réaction.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mis en oeuvre avec un système catalytique conforme à l'invention.

l'invention sera plus clairement illustrée au vu des exemples donnés ci-dessous à titre indicatif et illustratif.

Dans ces exemples, la signification des abréviations utilisées est donnée ci dessous :
PNP: 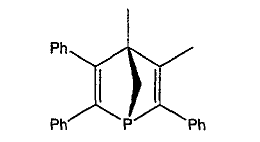
PNA: 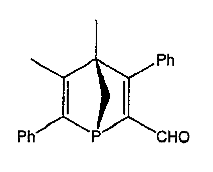
cod : 1,5-cyclooctadiène.
2M3BN : 2-méthyl-3-butène-nitrile.
2M2BN : 2-méthyl-2-butène-nitrile.
3PN : 3-pentènenitrile.
4PN : 4-pentènenitrile.
ADN : adiponitrile.
MGN : méthylglutaronitrile.
ESN : ethylsuccinonitrile.
DN : dinitriles = ADN + MGN + ESN.
CA : cyanhydrine de l'acétone.
EtPh : Ethylbenzène.
EG : Ethylèneglycol.
TT (V) : taux de transformation du composé V à hydrocyaner ou isomériser égal au rapport de la différence entre le nombre de moles engagées du composé V et le nombre de moles présentes en fin de réaction sur le nombre de moles engagées.
RR (U) : rendement réel du composé formé U = nb de moles formées de U / nb de mole maximale de U calculé par rapport au nombre de moles engagées du composé V.
RT (U) : sélectivité du composé U = RR (U) / TT (V).
L : Linéarité = RT(ADN) / [RT(ADN) + RT (MGN) + RT (ESN)].
CPG : chromatographie phase gazeuse.
mol : mole.
mmol : millimole.

### Exemples 1 et 2 : Isomérisation du 2M3BN en 3PN

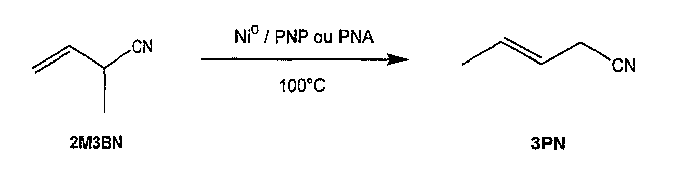

### Mode opératoire :

Dans un réacteur muni d'une agitation et mis sous atmosphère d'argon sont chargés 20 mg (0,073 mmol ; M = 275 g/mol ; 1,0 eq) de Ni(cod)₂ et 5,0 eq de ligand PNP ou PNA. Environ 1 ml (810 mg ; d = 0,81 ; M = 81,12 g/mol) de 2M3BN dégazé est ajouté. Le mélange est agité et maintenu à une température de 100°C en système fermé pendant 1 heure. Le milieu réactionnel est refroidi à température ambiante (eniron 20°C). Les concentrations des différents constituants du milieu réactionnel sont déterminées par analyse par CPG (Chromatographie en phase vapeur).

Les résultats obtenus et calculés à partir de ces analyses sont rassemblés dans la tableau I ci-dessous :

**Tableau I**

| **EX:** | **Ligand** | **Bilan molaire** | **TT (2M3BN)** | **RT(3+4PN)** | **RT (2M2BN)** |
|---|---|---|---|---|---|
| 1 | PNP | 97% | 87% | 89% | 6% |
| 2 | PNA | 97% | 48% | 84% | 8% |

### Exemples 3 à 5 : Hydrocyanation du 3PN en ADN

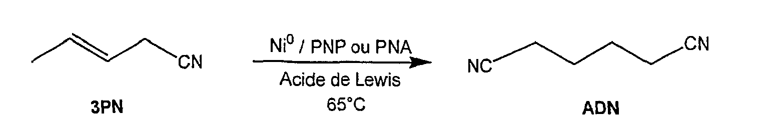

### Mode opératoire :

Dans un tube de Schlenk maintenu sous argon sont introduits successivement et à température ambiante : le ligand L (5eq), le 3PN (30 eq), le Ni(cod)₂ (1eq), le ZnCl₂ (1 eq), le ou les cosolvants dégazés et la cyanhydrine de l'acétone (30 eq). Le mélange est porté sous agitation (600 tr/min) à 65°C pendant 2 heures puis ramené à température ambiante. 3 ml d'acétone sont introduits pour neutraliser l'HCN restant. Les concentrations des différents composants sont déterminées par analyse CPG pour calculer les différents taux de transformation et de sélectivité.

Les résultats obtenus et calculés à partir de ces analyses sont rassemblés dans la tableau II ci-dessous :

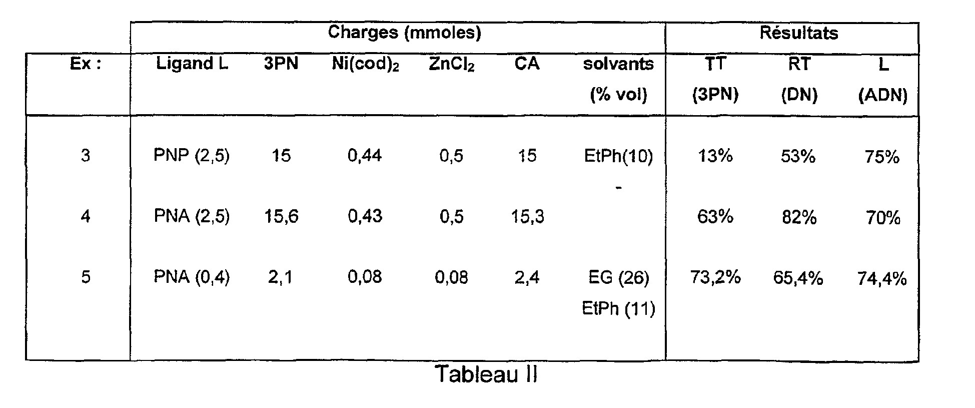

### Exemple 6 : Hydrocyanation du 3PN en ADN

### Mode opératoire :

Sous argon, dans un tube Shott de 20 ml muni d'un septum sont chargés 0,506 g (6,25 mmol ; M = 81 g/mol) de 3PN, 340 mg (0,93 mmol ; M = 366 g/mol) de PNP, 1,96 g de toluène dégazé, 56,5 mg (0,21 mmol ; M = 275 g/mol) de Ni(cod)₂ et 50,1 mg (0,21 mmol ; M = 242 g/mol) de BPh₃. Le mélange est porté sous agitation à 65°C et 531 mg (6,24 mmol ; M = 85 g/mol) de cyanhydrine de l'acétone sont injectés via le septum et à l'aide d'une pousse-seringue à un débit de 0,19 ml/h. Après 3h de réaction, le mélange est ramené à température ambiante et neutralisé pour éliminer l'HCN restant.. Les concentrations des différents composants sont déterminées par analyse CPG pour calculer les différents taux de transformation et de sélectivité.

Les résultats obtenus et calculés à partir de ces analyses sont rassemblés dans la tableau III ci-dessous :

**Tableau III**

| **EX:** | **TT (3PN)** | **RT (mononitriles)** | **RT (dinitriles)** | **Linéarité (ADN)** |
|---|---|---|---|---|
| 6 | 35% | 91,8% | 12,4% | 69% |

## Revendications

1. Procédé d'hydrocyanation de composés organiques comprenant au moins une liaison éthylénique par réaction avec le cyanure d'hydrogène, en présence d'un système catalytique comprenant un métal de transition et un ligand organophosphoré, **caractérisé en ce que** le ligand est une phosphine répondant à la formule générale (I) suivante : 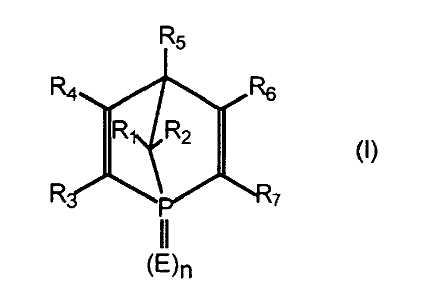
dans laquelle :
E représente O ou S ;
n représente 0 ou 1 ;
R₁, R₄, R₅ et R₆, identiques ou différents, représentent un atome d'hydrogène; ou bien un radical T choisi parmi : un radical hydrocarboné aliphatique, de 1 à 40 atomes de carbone, saturé ou insaturé, dont la chaîne hydrocarbonée est éventuellement interrompue par un hétéroatome; un radical carbocyclique ou hétérocyclique, saturé, insaturé ou aromatique, monocyclique ou polycyclique, ou un radical hydrocarboné aliphatique saturé ou insaturé dont la chaîne hydrocarbonée est éventuellement interrompue par un hétéroatome et porteur d'un radical carbocyclique ou hétérocyclique tel que défini ci-dessus,
ou bien R₄ et R₅ forment ensemble avec les atomes de carbone qui les portent un monocycle carbocyclique saturé ou insaturé ayant de préférence de 5 à 7 atomes de carbone ;
R₂ représente un atome d'hydrogène ou le radical X ;
R₃ représente le radical X ou le radical Y ;
étant entendu qu'un et un seul des substituants R₂ et R₃ représente le radical X ; X étant choisi parmi un radical carbocyclique ou hétérocyclique aromatique monocyclique ou bicyclique ayant de 2 à 20 atomes de carbone ; un radical 1-alcényle présentant éventuellement une ou plusieurs insaturations supplémentaires dans la chaîne hydrocarbonée et ayant de 2 à 12 atomes de carbone ; un radical 1-alcynyle présentant éventuellement une ou plusieurs insaturations supplémentaires dans la chaîne hydrocarbonée et ayant de 2 à 12 atomes de carbone ; un radical -CN ; [(C₁-C₁₂)alkyl]carbonyle ; [(C₃₋C₁₈)aryl]carbonyle ; [(C₁-C₁₂)alcoxy]carbonyle ; [(C₆-C₁₈)aryloxy]carbonyle ; carbamoyle ; [(C₁-C₁₂)alkyl]carbamoyle ; ou [di(C₁-C₁₂)alkyl]carbamoyle; et Y prenant l'une quelconque des significations de R₁ à l'exception d'un atome d'hydrogène ;
R₇ a la signification de R₁, R₄, R₅ et R₆ ou représente un radical hydrocarboné comprenant une fonction carbonyle ou un radical de formules suivantes : 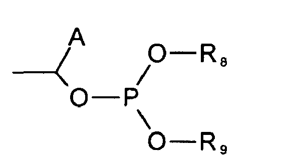
ou 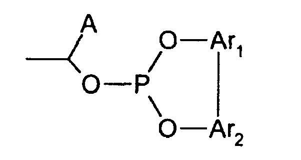
dans lesquelles,
A représente un atome d'hydrogène ; (C₁-C₁₀)alkyle ; ou (C₆-C₁₀)aryle ou (C₆-C₁₀)aryl-(C₁₋C₁₀)alkyle dans lesquels la partie aryle est éventuellement substituée par un ou plusieurs radicaux choisis parmi (C₁-C₆)alkyle, (C₁-C₆)alcoxy, trifluorométhyle, halogène, di(C₁-C₆)alkylamino, (C₁-C₆)alcoxycarbonyle, carbamoyle, (C₁₋C₆)alkylaminocarbonyle et di(C₁-C₆)alkylaminocarbonyle ;
-Ar₁ ; Ar₂- représentent :
• soit le radical divalent de formule: 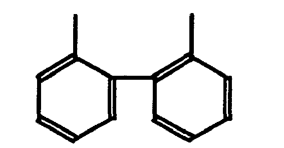
dans lequel chacun des noyaux phényle est éventuellement substitué par un ou plusieurs groupes Z tels que définis ci-dessous ;
• soit le radical divalent de formule : 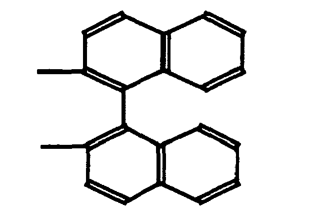
dans lequel chacun des noyaux phényle est éventuellement substitué par un ou plusieurs groupes Z tels que définis ci-dessous
Z représente (C₁-C₆)alkyle, (C₁-C₆)alcoxy, trifluorométhyle, halogène, (C₁₋C₆)alcoxycarbonyle, di (C₁-C₆)alkylamino, (C₁-C₆)alkylaminocarbonyle ou di(C₁₋C₆)alkylaminocarbonyle.
R8 et R9 identiques ou différents représentent un radical aryle.

2. Procédé selon la revendication 1, **caractérisé en ce que** les composés phosphines sont ceux répondant à la formule (I) dans laquelle :
R₁, R₄, R₅ et R₆ représentent indépendamment un atome d'hydrogène ou bien un radical T choisi parmi :
· un radical hydrocarboné aliphatique, saturé ou insaturé, ayant de 1 à 12 atomes de carbone, dont la chaîne hydrocarbonée est éventuellement interrompue par un hétéroatome choisis parmi O, N et S;
· un radical carbocyclique monocyclique saturé ou comprenant 1 ou 2 insaturations dans le cycle, ayant de 3 à 8 atomes de carbone;
· un radical carbocyclique bicyclique saturé ou insaturé constitué de 2 monocycles condensés l'un à l'autre, chaque monocycle comprenant éventuellement 1 à 2 insaturations et présentant de 3 à 8 atomes de carbone;
· un radical carbocyclique aromatique mono- ou bicyclique en C₆-C₁₀;
· un radical hétérocyclique monocyclique saturé, insaturé ou aromatique de 5 à 6 chaînons comprenant 1 à 3 hétéroatomes choisis indépendamment parmi N, O et S;
· un radical hétérocyclique bicyclique saturé, insaturé ou aromatique constitué de deux monocycles de 5 à 6 chaînons condensés l'un à l'autre, chaque monocycle comprenant 1 à 3 hétéroatomes choisis indépendamment parmi O, N et S; et
· un radical hydrocarboné aliphatique saturé ou insaturé, ayant de 1 à 12 atomes de carbone, dont la chaîne hydrocarbonée porte un radical monocyclique carbocyclique ou hétérocyclique tel que défini ci-dessus
R2 représentent indépendamment un atome d'hydrogène ou bien un radical X choisi parmi:
· un radical carbocyclique monocyclique saturé ou comprenant 1 ou 2 insaturations dans le cycle, ayant de 3 à 8 atomes de carbone;
· un radical carbocyclique bicyclique saturé ou insaturé constitué de 2 monocycles condensés l'un à l'autre, chaque monocycle comprenant éventuellement 1 à 2 insaturations et présentant de 3 à 8 atomes de carbone;
un radical carbocyclique aromatique mono- ou bicyclique en C₆-C₁₀;

3. Procédé selon la revendication 1 ou 2, **caractérisé en ce que** X est choisi parmi un groupe alcényle en (C₂-C₆), un groupe alcynyle en (C₂-C₆), phényle, naphtyle, thiényle, furyle, pyrrolyle, imidazolyle, pyrazolyle, thiazolyle, oxazolyle, pyridyle, pyrazinyle, pyridazinyle, isothiazolyle, isoxazolyle, benzofuryle, benzothiényle, indolyle, isoindolyle, indolizinyle, indazolyle, purinyle, quinolyle, isoquinolyle, benzoxazolyle, benzothiazolyle et ptéridinyle

4. Procédé selon l'une des revendications précédentes, **caractérisé en ce que** la phosphine répond soit à la formule II soit à la formule III suivantes :
Formule (II) : 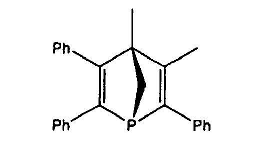
Formule (III) :

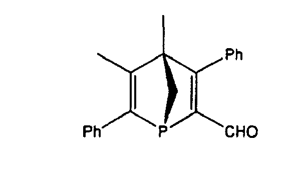

5. Procédé selon l'une des revendications précédentes, **caractérisé en ce que** les composés des métaux de transition sont choisis parmi les composés du nickel, du palladium et du fer.

6. Procédé selon l'une des revendications précédentes, **caractérisé en ce que** les composés des métaux de transition préférés sont ceux du nickel et sont choisis parmi :
les composés dans lesquel le nickel est au degré d'oxydation zéro comme le tétracyanonickelate de potassium K₄ [Ni(CN)₄], le bis(acrylonitrile) nickel zéro, le bis(cyclooctadiène-1,5) nickel et les dérivés contenant des ligands du groupe Va comme le tétrakis(triphényl-phosphine) nickel zéro ;
les composés du nickel comme les carboxylates, carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

7. Procédé selon l'une des revendications précédentes, **caractérisé en ce que** les composés organiques comportant au moins une double liaison éthylénique sont choisis parmi les dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3 nitrile, le pentène-4 nitrile, les monooléfines comme le styrène, le méthyl-styrène, le vinyl-naphtalène, le cyclohexène, le méthyl-cyclohexène ainsi que les mélanges de plusieurs de ces composés.

8. Procédé selon l'une des revendications précédentes, **caractérisé en ce que** la quantité de composé du nickel ou d'un autre métal de transition utilisée est choisie de telle sorte qu'il y ait par mole de composé organique à hydrocyaner ou isomériser entre 10⁻⁴ et 1 mole de nickel ou de l'autre métal de transition mis en oeuvre et **en ce que** la quantité de phosphine de formule (I) utilisée est choisie de telle sorte que le nombre de moles de ce composé rapporté à 1 mole de métal de transition soit de 0,5 à 500.

9. Procédé selon l'une des revendications précédentes, **caractérisé en ce que** la réaction d'hydrocyanation est réalisée à une température de 10°C à 200°C.

10. Procédé selon l'une des revendications précédentes d'hydrocyanation en dintriles de composés nitriles à insaturation éthylénique, par réaction avec le cyanure d'hydrogène, **caractérisé en ce que** l'on opère en présence d'un système catalytique comprenant au moins un composé d'un métal de transition, au moins une phosphine de formule (I) et un cocatalyseur consistant en au moins un acide de Lewis.

11. Procédé selon la revendication 10, **caractérisé en ce que** les composés nitriles à insaturation éthylénique sont choisis parmi les nitriles aliphatiques à insaturation éthylénique comprenant les pentène-nitriles linéaires comme le pentène-3 nitrile, le pentène-4 nitrile et leurs mélanges.

12. Procédé selon la revendication 11, **caractérisé en ce que** les pentène-nitriles linéaires contiennent des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène.

13. Procédé selon l'une des revendications 10 à 12, **caractérisé en ce que** l'acide de Lewis mis en oeuvre comme cocatalyseur est choisi parmi les composés des éléments des groupes Ib, IIb, IIIa, IIIb, IVa, IVb, Va, Vb, Vlb, VIIb et VIII de la Classification périodique des éléments.

14. Procédé selon l'une des revendications 10 à 13, **caractérisé en ce que** l'acide de Lewis est choisi parmi les sels choisi dans le groupe des halogénures, sulfates, sulfonates, halogenoalkylsulfonates, perhalogénoalkyl sulfonates, carboxylates et phosphates.

15. Procédé selon l'une des revendications 10 à 14, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium, le chlorure de cobalt, le chlorure ferreux, le chlorure d'yttrium et leurs mélanges.

16. Procédé selon l'une des revendications 10 à 15, **caractérisé en ce que** l'acide de Lewis mis en oeuvre représente de 0,01 à 50 moles par mole de composé de métal de transition.

17. Procédé selon l'une des revendications 1 à 16, **caractérisé en ce que** l'on réalise en absence de cyanure d'hydrogène l'isomérisation en pentène-nitriles duméthyl-2 butène-3 nitrile présent dans le mélange réactionnel provenant de l'hydrocyanation du butadiène, en opérant en présence d'un catalyseur comportant au moins une phosphine de formule (I) et au moins un composé d'un métal de transition.

18. Procédé selon la revendication 17, **caractérisé en ce que** le méthyl-2 butène-3 nitrile soumis à l'isomérisation est mis en oeuvre seul ou en mélange avec du méthyl-2 butène-2 nitrile, du pentène-4 nitrile, du pentène-3 nitrile, du pentène-2 nitrile, du butadiène, de l'adiponitrile, du méthyl-2 glutaronitrile, de l'éthyl-2 succinonitrile ou du valéronitrile.

19. Procédé selon l'une des revendications 17 ou 18, **caractérisé en ce que** la réaction d'isomérisation est réalisée à une température de 10°C à 200°C.

20. Procédé selon l'une des revendications 17 à 19, **caractérisé en ce que** l'isomérisation en pentène-nitriles du méthyl-2 butène-3 nitrile est réalisée en présence d'au moins un composé d'un métal de transition, d'au moins une phosphine de formule (I) et un cocatalyseur consistant en au moins un acide de Lewis.

## Patentansprüche

1. Verfahren zur Hydrocyanierung von organischen Verbindungen, die mindestens eine ethylenische Bindung umfassen, durch Reaktion mit Cyanwasserstoff in Anwesenheit eines katalytischen Systems, das ein Übergangsmetall und einen Organophosphor-Liganden umfaßt, **dadurch gekennzeichnet, daß** der Ligand ein Phosphin ist, das der folgenden allgemeinen Formel (I) entspricht: 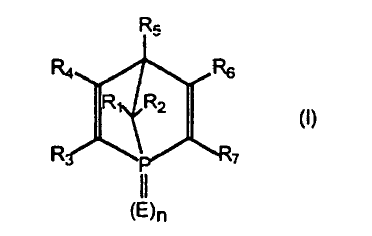
in der
E O oder S darstellt;
n 0 oder 1 darstellt;
R₁, R₄, R₅ und R₆, gleich oder verschieden, ein Wasserstoffatom oder auch einen Rest T darstellen, ausgewählt unter: einem gesättigten oder ungesättigten aliphatischen Kohlenwasserstoff-Rest mit 1 bis 40 Kohlenstoffatomen, dessen Kohlenwasserstoff-Kette gegebenenfalls durch ein Heteroatom unterbrochen ist; einem gesättigten, ungesättigten oder aromatischen, monocyclischen oder polycyclischen, carbocyclischen oder heterocyclischen Rest, oder einem gesättigten oder ungesättigten aliphatischen Kohlenwasserstoff-Rest, dessen Kohlenwasserstoff-Kette gegebenenfalls durch ein Heteroatom unterbrochen ist und der einen wie oben definierten carbocyclischen oder heterocyclischen Rest trägt,
oder auch R₄ und R₅ zusammen mit den Kohlenstoffatomen, die sie tragen, einen gesättigten oder ungesättigten carbocyclischen Monocyclus mit vorzugsweise 5 bis 7 Kohlenstoffatomen bilden;
R₂ ein Wasserstoffatom oder den Rest X darstellt;
R₃ den Rest X oder den Rest Y darstellt;
mit der Maßgabe, daß einer und ein alleinstehender der Substituenten R₂ und R₃ den Rest X darstellt;
X ausgewählt wird unter einem aromatischen, monocyclischen oder bicyclischen, carbocyclischen oder heterocyclischen Rest mit 2 bis 20 Kohlenstoffatomen; einem Rest 1-Alkenyl, der gegebenenfalls eine oder mehrere zusätzliche Unsättigungen in der Kohlenwasserstoff-Kette aufweist und 2 bis 12 Kohlenstoffatome besitzt; einem Rest 1-Alkinyl, der gegebenenfalls eine oder mehrere zusätzliche Unsättigungen in der Kohlenwasserstoff-Kette aufweist und 2 bis 12 Kohlenstoffatome besitzt; einem Rest -CN;
[(C₁-C₁₂)Alkyl]-carbonyl; [(C₃-C₁₈)Aryll-carbonyl; [(C₁-C₁₂)Alkoxy] - carbonyl; [(C₆-C₁₈)Aryloxy]-carbonyl; Carbamoyl; [(C₁-C₁₂)Alkyl] - carbamoyl; oder [Di-(C₁-C₁₂)alkyl]-carbamoyl; und
Y irgendeine der Bedeutungen von R₁ annimmt, mit Ausnahme eines Wasserstoffatoms;
R₇ die Bedeutung von R₁, R₄, R₅ und R₆ besitzt oder einen Kohlenwasserstoff-Rest darstellt, der eine Carbonylfunktion oder einen Rest der folgenden Formeln umfaßt: 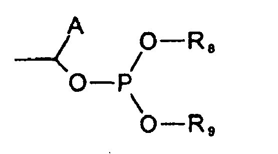
oder 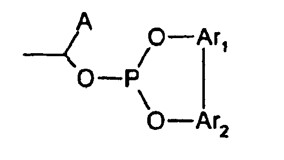
in denen
A ein Wasserstoffatom; (C₁-C₁₀)Alkyl; oder (C₆-C₁₀)Aryl; oder (C₆-C₁₀)Aryl-(C₁-C₁₀)alkyl darstellt; worin der Teil Aryl gegebenenfalls substituiert ist durch einen oder mehrere Reste, ausgewählt unter (C₁-C₆)Alkyl, (C₁-C₆)Alkoxy, Trifluormethyl, Halogen, Di- (C₁-C₆) alkylamino, (C₁-C₆) Alkoxycarbonyl, Carbamoyl, (C₁-C₆) - Alkylaminocarbonyl und Di-(C₁-C₆)alkylaminocarbonyl;
-Ar₁ ; Ar₂- darstellen:
· entweder den divalenten Rest der Formel 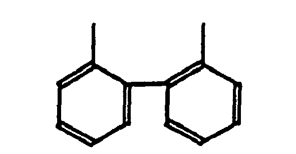
worin jeder der Phenylkerne gegebenenfalls substituiert ist durch eine oder mehrere Gruppen Z wie nachstehend definiert,
· oder den divalenten Rest der Formel 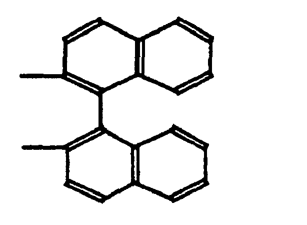
worin jeder der Phenylkerne gegebenenfalls substituiert ist durch eine oder mehrere Gruppen Z wie nachstehend definiert, Z (C₁-C₆)Alkyl, (C₁-C₆)Alkoxy, Trifluormethyl, Halogen, (C₁-C₆) Alkoxycarbonyl, Di-(C₁-C₆)alkylamino, (C₁-C₆)-Alkylaminocarbonyl oder Di-(C₁-C₆)alkylaminocarbonyl darstellt; und
R₈ und R₉, gleich oder verschieden, einen Rest Aryl bedeuten.

2. Verfahren nach Anspruch 1, **dadurch gekennzeichnet, daß** die Phosphin-Verbindungen solche sind, die der allgemeinen Formel (I) entsprechen, in der
R₁, R₄, R₅ und R₆ unabhängig ein Wasserstoffatom oder auch einen Rest T darstellen, ausgewählt unter:
einem gesättigten oder ungesättigten aliphatischen Kohlenwasserstoff-Rest mit 1 bis 12 Kohlenstoffatomen, dessen Kohlenwasserstoff-Kette gegebenenfalls durch ein Heteroatom unterbrochen ist, ausgewählt unter O, N und S;
einem monocyclischen carbocyclischen Rest, gesättigt oder ein oder zwei Unsättigungen im Ring umfassend, mit 3 bis 8 Kohlenstoffatomen;
einem gesättigten oder ungesättigten bicyclischen carbocyclischen Rest, bestehend aus zwei Monocyclen, die miteinander kondensiert sind, wobei jeder Monocyclus gegebenenfalls ein oder zwei Unsättigungen umfaßt und 3 bis 8 Kohlenstoffatome aufweist;
einem mono- oder bicyclischen, aromatischen carbocyclischen Rest mit 6 bis 10 Kohlenstoffatomen,
einem gesättigten, ungesättigten oder aromatischen monocyclischen heterocyclischen Rest mit 5 bis 6 Kettengliedern, umfassend 1 bis 3 Heteroatome, ausgewählt unter N, 0 und S;
einem gesättigten, ungesättigten oder aromatischen bicyclischen heterocyclischen Rest, bestehend aus zwei Monocyclen mit 5 bis 6 Kettengliedern, die miteinander kondensiert sind, wobei jeder Monocyclus 1 bis 3 Heteroatome umfaßt, ausgewählt unter O, N und S; und einem gesättigten oder ungesättigten aliphatischen Kohlenwasserstoff-Rest mit 1 bis 12 Kohlenstoffatomen, dessen Kohlenwasserstoff-Kette einen wie oben definierten carbocyclischen oder heterocyclischen monocyclischen Rest trägt,
R₂ unabhängig ein Wasserstoffatom oder auch einen Rest X darstellt, ausgewählt unter:
einem monocyclischen carbocyclischen Rest, gesättigt oder umfassend ein oder zwei Unsättigungen im Ring, mit 3 bis 8 Kohlenstoffatomen;
einem gesättigten oder ungesättigten bicyclischen carbocyclischen Rest, bestehend aus zwei Monocyclen, die miteinander kondensiert sind, wobei jeder Monocyclus gegebenenfalls ein bis 2 Unsättigungen umfaßt und 3 bis 8 Kohlenstoffatome aufweist;
einem mono- oder bicyclischen, aromatischen carbocyclischen Rest mit 6 bis 10 Kohlenstoffatomen.

3. Verfahren nach Anspruch 1 oder 2, **dadurch gekennzeichnet, daß** X ausgewählt wird unter einer Gruppe Alkenyl mit 2 bis 6 Kohlenstoffatomen, einer Gruppe Alkinyl mit 2 bis 6 Kohlenstoffatomen, Phenyl, Naphthyl, Thienyl, Furyl, Pyrrolyl, Imidazolyl, Pyrazolyl, Thiazolyl, Oxazolyl, Pyridyl, Pyrazinyl, Pyridazinyl, Isothiazolyl, Isoxazolyl, Benzofuryl, Benzothienyl, Indolyl, Isoindolyl, Indolizinyl, Indazolyl, Purinyl, Chinolyl, Isochinolyl, Benzoxazolyl, Benzothiazolyl und Pteridinyl.

4. Verfahren nach einem der vorstehenden Ansprüche, **dadurch gekennzeichnet, daß** das Phosphin entweder der Formel (II) oder der Formel (III) entspricht, die folgen:
Formel (II) 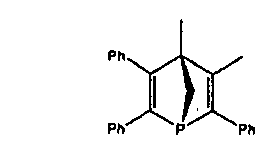
Formel (III)

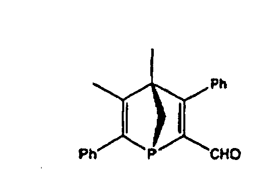

5. Verfahren nach einem der vorstehenden Ansprüche, **dadurch gekennzeichnet, daß** die Verbindungen der Übergangsmetalle unter den Verbindungen von Nickel, Palladium und Eisen ausgewählt werden.

6. Verfahren nach einem der vorstehenden Ansprüche, **dadurch gekennzeichnet, daß** die bevorzugten Verbindungen der Übergangsmetalle diejenigen von Nickel sind und ausgewählt werden unter:
den Verbindungen, in denen das Nickel den Oxidationsgrad null besitzt, wie Kalium-tetracyanonickelat K₄[Ni(CN)₄], Bis-(Acrylonitril)-Nickel null, Bis-(1,5-Cyclooctadien)-Nickel und die Derivate, die Liganden der Gruppe Va enthalten wie Tetrakis-(Triphenylphosphin)-Nickel null;
den Verbindungen von Nickel wie Carboxylate, Carbonat, Bicarbonat, Borat, Bromid, Chlorid, Citrat, Thiocyanat, Cyanid, Formiat, Hydroxid, Hydrophosphit, Phosphit, Phosphat und Derivate, Iodid, Nitrat, Sulfat, Sulfit, Aryl- und Alkylsulfonate.

7. Verfahren nach einem der vorstehenden Ansprüche, **dadurch gekennzeichnet, daß** die organischen Verbindungen, die mindestens eine ethylenische Doppelbindung umfassen, ausgewählt werden unter den Diolefinen wie Butadien, Isopren, 1,5-Hexadien, 1,5-Cyclooctadien, den aliphatischen Nitrilen mit ethylenischer Unsättigung, insbesondere den linearen Penten-nitrilen wie 3-Penten-nitril, 4-Pentennitril, den Monoolefinen wie Styrol, Methylstyrol, Vinylnaphthalin, Cyclohexen, Methylcyclohexen sowie den Mischungen von mehreren dieser Verbindungen.

8. Verfahren nach einem der vorstehenden Ansprüche, **dadurch gekennzeichnet, daß** die Menge der verwendeten Verbindung von Nickel oder von einem anderen Übergangsmetall in der Weise ausgewählt wird, daß pro mol organischer Verbindung, die zu hydrocyanieren oder zu isomerisieren ist, zwischen 10⁻⁴ und 1 mol eingesetztes Nickel oder anderes Übergangsmetall vorliegen, und daß die Menge von verwendetem Phosphin der Formel (I) in der Weise ausgewählt wird, daß die Anzahl von Molen dieser Verbindung, bezogen auf 1 mol Übergangsmetall, 0,5 bis 500 beträgt.

9. Verfahren nach einem der vorstehenden Ansprüche, **dadurch gekennzeichnet, daß** die Reaktion der Hydrocyanierung bei einer Temperatur von 10 °C bis 200 °C realisiert wird.

10. Verfahren nach einem der vorstehenden Ansprüche zur Hydrocyanierung von Nitrilverbindungen mit ethylenischer Unsättigung zu Dinitrilen durch Reaktion mit Cyanwasserstoff, **dadurch gekennzeichnet, daß** man in Anwesenheit eines katalytischen Systems arbeitet, das mindestens eine Verbindung eines Übergangsmetalls, mindestens ein Phosphin der Formel (I) und einen Co-Katalysator umfaßt, der aus mindestens eine Lewis-Säure besteht.

11. Verfahren nach Anspruch 10, **dadurch gekennzeichnet, daß** die Nitrilverbindungen mit ethylenischer Unsättigung ausgewählt werden unter den aliphatischen Nitrilen mit ethylenischer Unsättigung, umfassend die linearen Penten-nitrile wie 3-Penten-nitril, 4-Penten-nitril und ihre Mischungen.

12. Verfahren nach Anspruch 11, **dadurch gekennzeichnet, daß** die linearen Penten-nitrile im allgemeinen in der Minderheit vorliegende Mengen von anderen Verbindungen enthalten wie 2-Methyl-3-buten-nitril, 2-Methyl-2-buten-nitril, 2-Penten-nitril, Valeronitril, Adiponitril, 2-Methyl-glutaro-nitril, 2-Ethyl-succino-nitril oder Butadien.

13. Verfahren nach einem der Ansprüche 10 bis 12, **dadurch gekennzeichnet, daß** die als Co-Katalysator eingesetzte Lewis-Säure ausgewählt wird unter den Verbindungen der Elemente der Gruppen Ib, IIb, IIIa, IIIb, IVa, IVb, Va, Vb, VIb, VIIb und VIII des Periodensystems der Elemente.

14. Verfahren nach einem der Ansprüche 10 bis 13, **dadurch gekennzeichnet, daß** die Lewis-Säure unter den Salzen ausgewählt wird, gewählt aus der Gruppe der Halogenide, Sulfate, Sulfonate, Halogenalkylsulfonate, Perhalogenalkylsulfonate, Carboxylate und Phosphate.

15. Verfahren nach einem der Ansprüche 10 bis 14, **dadurch gekennzeichnet, daß** die Lewis-Säure ausgewählt wird unter Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indium-trifluormethylsulfonat, den Chloriden oder Bromiden der Elemente der Seltenen Erden wie Lanthan, Cerium, Praseodym, Neodym, Samarium, Europium, Gadolinium, Terbium, Dysprosium, Holmium, Erbium, Thulium, Ytterbium und Lutetium, Cobaltchlorid, Eisen(II)-chlorid, Yttriumchlorid und ihren Mischungen.

16. Verfahren nach einem der Ansprüche 10 bis 15, **dadurch gekennzeichnet, daß** die eingesetzte Lewis-Säure 0,01 bis 50 mol pro mol der Verbindung des Übergangsmetalls darstellt.

17. Verfahren nach einem der Ansprüche 1 bis 16, **dadurch gekennzeichnet, daß** man bei Abwesenheit von Cyanwasserstoff die Isomerisierung von 2-Methyl-3-buten-nitril, das in der Reaktionsmischung vorliegt, die von der Hydrocyanierung von Butadien stammt, zu Penten-nitrilen realisiert, indem man in Anwesenheit eines Katalysators arbeitet, der mindestens ein Phosphin der Formel (I) und mindestens eine Verbindung eines Übergangsmetalls umfaßt.

18. Verfahren nach Anspruch 17, **dadurch gekennzeichnet, daß** das 2-Methyl-3-buten-nitril einer Isomerisierung unterzogen und allein oder in Mischung mit 2-Methyl-2-buten-nitril, 4-Penten-nitril, 3-Penten-nitril, 2-Penten-nitril, Butadien, Adiponitril, 2-Methyl-glutaro-nitril, 2-Ethyl-succino-nitril oder Valeronitril eingesetzt wird.

19. Verfahren nach einem der Ansprüche 17 oder 18, **dadurch gekennzeichnet, daß** die Reaktion der Isomerisierung bei einer Temperatur von 10 °C bis 200 °C realisiert wird.

20. Verfahren nach einem der Ansprüche 17 bis 19, **dadurch gekennzeichnet, daß** die Isomerisierung von 2-Methyl-3-buten-nitril zu Penten-nitrilen in Anwesenheit von mindestens einer Verbindung eines Übergangsmetalls, von mindestens einem Phosphin der Formel (I) und einem Co-Katalysator realisiert wird, der aus mindestens einer Lewis-Säure besteht.

## Claims

1. Process for the hydrocyanation of organic compounds comprising at least one ethylenic bond by reaction with hydrogen cyanide in the presence of a catalytic system comprising a transition metal and an organophosphorus ligand, **characterized in that** the ligand is a phophine corresponding to the following general formula (I) : 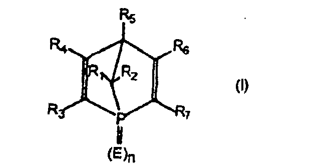
in which:
E represents O or S;
n represents 0 or 1;
R₁, R₄, R₅ and R₆, which are identical or different, represent a hydrogen atom; or else a T radical chosen from: a saturated or unsaturated, aliphatic hydrocarbonaceous radical comprising 1 to 40 carbon atoms, the hydrocarbonaceous chain of which is optionally interrupted by a heteroatom; a monocyclic or polycyclic, saturated, unsaturated or aromatic, carbocyclic or heterocyclic radical; or a saturated or unsaturated, aliphatic hydrocarbonaceous radical, the hydrocarbonaceous chain of which is optionally interrupted by a heteroatom and carries a carbocyclic or heterocyclic radical as defined above;
or else R4 and R₅ form, together with the carbon atoms which carry them, a saturated or unsaturated, carbocyclic monocycle preferably having from 5 to 7 carbon atoms;
R₂ represents a hydrogen atom or the X radical;
R₃ represents the X radical or the Y radical;
it being understood that one and one alone of the R₂ and R₃ substituents represents the X radical;
X being chosen from a monocyclic or bicyclic, aromatic carbocyclic or heterocyclic radical having from 2 to 20 carbon atoms; a 1-alkenyl radical optionally exhibiting one or more additional unsaturations in the hydrocarbonaceous chain and having from 2 to 12 carbon atoms; a 1-alkynyl radical optionally exhibiting one or more additional unsaturations in the hydrocarbonaceous chain and having from 2 to 12 carbon atoms; or a -CN, [ (C₁-C₁₂) alkyl] carbonyl, [(C₃-C₁₈)aryl] carbonyl , [(C₁-C₁₂)alkoxy] carbonyl, [(C₆-C₁₈)aryloxy]carbonyl, carbamoyl, [(C₁-C₁₂)alkyl]] carbamoyl or [di(C₁-C₁₂)alkyl]carbamoyl radical; and
Y taking any one of the meanings of R₁, with the exception of a hydrogen atom;
R₇ has the meaning of R₁, R₄, R₅ and R₆ or represents a hydrocarbonaceous radical comprising a carbonyl functional group or a radical of following formulae: 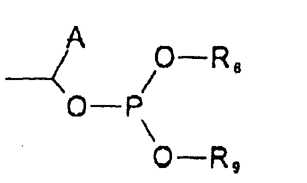
or 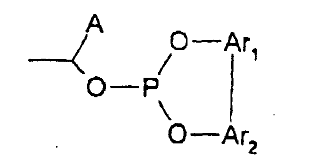
in which,
A represents a hydrogen atom; (C₁-C₁₀)alkyl; or (C₆-C₁₀)aryl or (C₆-C₁₀)aryl(C₁-C₁₀)alkyl in which the aryl part is optionally substituted by one or more radicals chosen from (C₁-C₆)alkyl, (C₁-C₆)alkoxy, trifluoromethyl, halogen, di (C₁-C₆)alkylamino, (C₁-C₆)alkoxycarbonyl, carbamoyl, (C₁-C₆) alkylaminocarbonyl and diC₁-C₆)alkylaminocarbonyl;
-Ar₁-Ar₂- represent:
• either the divalent radical of formula: 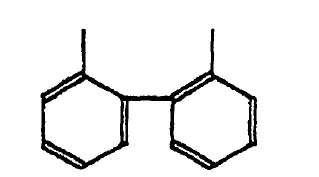
in which each of the phenyl nuclei is optionally substituted by one or more Z groups as defined below;
• or the divalent radical of formula: 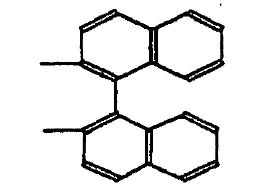
in which each of the phenyl nuclei is optionally substituted by one or more Z groups as defined below;
Z represents (C₁-C₆)alkyl, (C₁-C₆)alkoxy, trifluoromethyl, halogen, (C₁-C₆)alkoxycarbonyl, di(C₁-C₆)alkylamino, (C₁-C₆)alkylaminocarbonyl or di(C₁-C₆) alkylaminocarbonyl;
R₈ and R₉, which are identical or different, represent an aryl radical.

2. Process according to claim 1, **characterized in that** the phosphine compounds are those corresponding to the formula (I) in which:
R₁, R₄, R₅ and R₆ independently represent a hydrogen atom or else a T radical chosen from:
. a saturated or unsaturated aliphatic hydrocarbonaceous radical having from 1 to 12 carbon atoms, the hydrocarbonaceous chain of which is optionally interrupted by a heteroatom chosen from O, N and S;
. a monocyclic carbocyclic radical which is saturated or which comprises 1 or 2 unsaturations in the ring, having from 3 to 8 carbon atoms;
. a saturated or unsaturated bicyclic carbocyclic radical composed of 2 single rings condensed to one another, each single ring optionally comprising 1 to 2 unsaturations and exhibiting from 3 to 8 carbon atoms;
. a mono- or bicyclic C₆-C₁₀ aromatic carbocyclic radical;
. a saturated, unsaturated or aromatic 5- to 6-membered monocyclic heterocyclic radical comprising 1 to 3 heteroatoms chosen independently from N, 0 and S;
. a saturated, unsaturated or aromatic bicyclic heterocyclic radical composed of two 5- to 6-membered single rings condensed to one another, each single ring comprising 1 to 3 heteroatoms chosen independently from O, N and S; and
. a saturated or unsaturated aliphatic hydrocarbonaceous radical having from 1 to 12 carbon atoms, the hydrocarbonaceous chain of which carries a carbocyclic or heterocyclic monocyclic radical as defined above;
R₂ represents a hydrogen atom or else an X radical chosen from:
· a monocyclic carbocyclic radical which is saturated or which comprises 1 or 2 unsaturations in the ring, having from 3 to 8 carbon atoms;
· a saturated or unsaturated bicyclic carbocyclic radical composed of 2 single rings condensed to one another, each single ring optionally comprising 1 to 2 unsaturations and exhibiting from 3 to 8 carbon atoms;
· a mono- or bicyclic C₆-C₁₀ aromatic carbocyclic radical.

3. Process according to claim 1 2, **characterized in that** X is chosen from a (C₂-C₆)alkenyl group, a (C₂-C₆)alkynyl group, phenyl, naphthyl, thienyl, furyl, pyrrolyl, imidazolyl, pyrazolyl, thiazolyl, oxazolyl, pyridyl, pyrazinyl, pyridazinyl, isothiazolyl, isoxazolyl, benzofuryl, benzothienyl, indolyl, isoindolyl, indolizinyl, indazolyl, purinyl, quinolyl, isoquinolyl, benzoxazolyl, benzothiazolyl and pteridinyl.

4. Process according to one of the preceding claims, **characterized in that** the phosphine corresponds either to the following formula II or to the following formula III:
Formula (II): 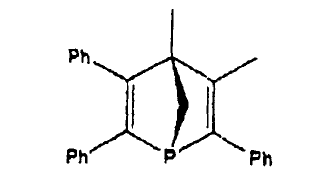
Formula (III):

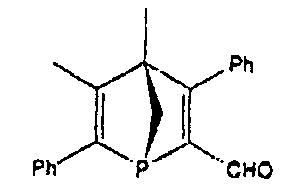

5. Process according to one of the preceding claims, **characterized in that** the transition metal compounds are chosen from nickel, palladium and iron compounds.

6. Process according to one of the preceding claims, **characterized in that** the preferred transition metal compounds are those of nickel and are chosen from:
compounds in which the nickel is in the zero oxidation state, such as potassium tetracyanonickelate K₄[(Ni(CN)₄], bis (acrylonitrile) nickel (0),
bis (1,5-cyclooctadiene) nickel and the derivatives comprising ligands from Group Va, such as tetrakis (triphenylphosphine) nickel (0);
nickel compounds, such as the carboxylates, carbonate, bicarbonate, borate, bromide, chloride, citrate, thiocyanate, cyanide, formate, hydroxide, hydrophosphite, phosphite, phosphate and derivatives, iodide, nitrate, sulphate, sulphite, arylsulphonates and alkylsulphonates.

7. Process according to one of the preceding claims, **characterized in that** the organic compounds comprising at least one ethylenic double bond are chosen from diolefins, such as butadiene, isoprene, 1,5-hexadiene or 1,5-cyclooctadiene, aliphatic nitriles comprising ethylenic unsaturation, particularly linear pentenenitriles, such as 3-pentenenitrile or 4-pentenenitrile, monoolefins, such as styrene, methylstyrene, vinylnaphthalene, cyclohexene or methylcyclohexene, and the mixtures of several of these compounds.

8. Process according to one of the preceding claims, **characterized in that** the amount of nickel compound or compound of another transition metal used is chosen so that there is, per mole of organic compound to be hydrocyanated or isomerized, between 10⁻⁴ and 1 mol of nickel or of the other transition metal employed and **in that** the amount of phosphine of formula (I) used is chosen so that the number of moles of this compound with respect to 1 mol of transition metal is from 0.5 to 500.

9. Process according to one of the preceding claims, **characterized in that** the hydrocyanation reaction is carried out at a temperature of 10°C to 200°C.

10. Process according to one of the preceding claims for the hydrocyanation to dinitriles of nitrile compounds comprising ethylenic unsaturation by reaction with hydrogen cyanide, **characterized in that** the reaction is carried out in the presence of a catalytic system comprising at least one compound of a transition metal, at least one phosphine of formula (I) and a cocatalyst comprising at least one Lewis acid.

11. Process according to claim 10, **characterized in that** the nitrile compounds comprising ethylenic unsaturation are chosen from aliphatic nitriles comprising ethylenic unsaturation comprising linear pentenenitriles, such as 3-pentenenitrile, 4-pentenenitrile and their mixtures.

12. Process according to claim 11, **characterized in that** the linear pentenenitriles comprise amounts, generally minor amounts, of other compounds, such as 2-methyl-3-butenenitrile, 2-methyl-2-butenenitrile, 2-pentenenitrile, valeronitrile, adiponitrile, 2-methylglutaronitrile, 2-ethylsuccinonitrile or butadiene.

13. Process according to one of claims 10 to 12, **characterized in that** the Lewis acid employed as cocatalyst is chosen from the compounds of the elements from Groups Ib, IIb, IIIa, IIIb, IVa, IVb, Va, Vb, VIb, VIIb and VIII of the Periodic Table.

14. Process according to one of claims 10 to 13, **characterized in that** the Lewis acid is chosen from the salts chosen from the group of the halides, sulphates, sulphonates, haloalkylsulphonates, perhaloalkylsulphonates, carboxylates and phosphates.

15. Process according to one of claims 10 to 14, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulphate, stannous tartrate, indium trifluoromethylsulphonate, the chlorides or bromides of rare-earth elements, such as lanthanum, cerium, praseodymium, neodymium, samarium, europium, gadolinium, terbium, dysprosium, hofmium, erbium, thulium, ytterbium and lutetium, cobalt chloride, ferrous chloride, yttrium chloride and their mixtures.

16. Process according to one of claims 10 to 15, **characterized in that** the Lewis acid employed represents from 0.01 to 50 mol per mole of transition metal compound.

17. Process according to one of claims 1 to 16, **characterized in that** the isomerization to pentenenitriles of the 2-methyl-3-butenenitrile present in the reaction mixture originating from the hydrocyanation of butadiene is carried out in the absence of hydrogen cyanide, the isomerization being carried out in the presence of a catalyst comprising at least one phosphine of formula (I) and at least one compound of a transition metal.

18. Process according to claim 17, **characterized in that** the 2-methyl-3-butenenitrile subjected to isomerization is employed alone or as a mixture with 2-methyl-2-butenenitrile, 4-pentenenitrile, 3-pentenenitrile, 2-pentenenitrile, butadiene, adiponitrile, 2-methylglutaroronitrile, 2-ethylsuccinonitrile or valeronitrile.

19. Process according to either of claims 17 and 18, **characterized in that** the isomerization reaction is carried out at a temperature of 10°C to 200°C.

20. Process according to one of claims 17 to 19, **characterized in that** the isomerization to pentenenitriles of 2-methyl-3-butenenitrile is carried out in the presence of at least one compound of a transition metal, of at least one phosphine of formula (I) and a cocatalyst comprising at least one Lewis acid.
